(19) Europäisches Patentamt
European Patent Office
Office européen des brevets

(11) Veröffentlichungsnummer: **0 230 598 B1**

(12) # EUROPÄISCHE PATENTSCHRIFT

(45) Veröffentlichungstag der Patentschrift: **06.03.91**

(51) Int. Cl.[5]: **A01N 43/16**

(21) Anmeldenummer: **86117412.6**

(22) Anmeldetag: **15.12.86**

(54) **Verwendung von langkettigen Ethern in Pflanzenschutzmitteln.**

(30) Priorität: **23.12.85 DE 3545908**

(43) Veröffentlichungstag der Anmeldung:
**05.08.87 Patentblatt 87/32**

(45) Bekanntmachung des Hinweises auf die Patenterteilung:
**06.03.91 Patentblatt 91/10**

(84) Benannte Vertragsstaaten:
**AT BE CH DE ES FR GB IT LI NL**

(56) Entgegenhaltungen:
**FR-A- 2 302 685**
**GB-A- 2 166 974**
**US-A- 4 335 236**

**CHEMICAL ABSTRACTS, Band 90, Nr. 11, 12. März 1979, Seite 164, Zusammenfassung Nr. 82069z, Columbus, Ohio, US; K. SLAMA et al.: "Juvenile hormone activity of some glycosidic juvenogens", & HOPPE-SEYLER'S Z. PHYSIOL. CHEM. 1978, 359(10), 1407-12**

**CENTRAL PATENTS INDEX, Basic Abstracts Journal, Section C, Woche 8526;, 21. August 1985, Zusammenfassung Nr. 85-156869, Derwent Publications Ltd., London, GB; & JP-A-60 89 402 (YOSHITOMI PHARM IND KK)**

**20-05-1985**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Schröder, Peter, Dr.**
**Gerretsfeld 5**
**W-4060 Viersen(DE)**
Erfinder: **Bouten, Hans**
**Sebastianusweg 15**
**W-4170 Geldern 3(DE)**
Erfinder: **Biermann, Manfred, Dr.**
**Marktscheiderhof 25**
**W-4330 Mülheim(DE)**

EP 0 230 598 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Zum Schutz von Nutz- und Zierpflanzen vor Schädlingen, Krankheiten und Unkräutern werden heute verschiedenste Verfahren, angefangen von der rein mechanischen Entfernung von Unkräutern und Schädlingen bis hin zu gezielten Veränderungen der pflanzlichen Erbsubstanz, angewandt. Die am häufigsten anzutreffende Methode ist aber nach wie vor der Einsatz chemischer Bekämpfungsmittel, da auf diese Weise ein Erfolg meist einfacher und schneller als mit anderen Verfahren zu erzielen ist.

Nachteilig beim Einsatz von chemischen Wirkstoffen sind aber oft unerwünschte Nebenwirkungen, die beispielsweise auf hohe Toxizität oder mangelnde Abbaubarkeit zurückgehen können, und die oft nach kurzer Zeit eintretende Resistenzbildung bei Schädlingen. Das Bedürfnis, diese Nachteile zu vermeiden, bildet heute einen nahezu ebenso starken Antrieb bei der Suche nach neuen Wirkstoffen wie der Wunsch, stärkere und selektivere Stoffe aufzufinden.

Der Wunsch nach Wirkstoffen mit günstigem Eigenschaftsprofil war auch Ausgangspunkt der Entwicklungen, die zur vorliegenden Erfindung führten. Der Erfindung selbst liegt der Befund zugrunde, daß gewisse langkettige Alkylglycoside, die zum Teil als Tenside bekannt waren, auch auf dem Gebiet des Pflanzenschutzes vorteilhafte Eigenschaften aufweisen.

Gegenstand der Erfindung ist daher die Verwendung von Alkylglycosiden der allgemeinen Formel I

$H(\text{-Glyk})_n\text{-R} \times (EO)_m$ (I)

in der (-Glyk) den zweibindigen Rest eines Monosaccharids, n einen Wert zwischen 1 und 6, R eine langkettige primäre glycosidisch gebundene Alkyl- oder Alkenylgruppe mit 8 - 22 C-Atomen, EO die Ethylenglykolethergruppe und m einen Wert von 0 bis 100 darstellen, als Schadorganismen bekämpfenden Wirkstoff in Mitteln zum Schutz von Nutz- und Zierpflanzen.

Diese Alkylglycoside zeichnen sich durch ein ungewöhnlich breites Wirkungsspektrum gegen Schadorganismen aus und können die Wirkung einer Vielzahl von bekannten Pflanzenschutzmitteln und Herbiziden in unerwarteter Weise ergänzen oder verstärken. Dabei unterscheidet sich die Wirkung der Alkylglycoside grundlegend von der Wirkung anderer Tenside, die als Hilfsmittel üblicherweise bei der Ausbringung von Agrarchemikalien, z. B. als Emulgatoren oder Spreitungsmittel, eingesetzt werden.

Die erfindungsgemäßen Alkylglycoside besitzen sowohl fungizide als auch insektizide und akarizide Wirkung und lassen sich auf Grund dieser Eigenschaften als alleinige Wirkstoffe in den Mitteln zur Bekämpfung von Schädlingen einsetzen. Bei dieser Form der Anwendung kommen die geringe Toxizität und der gute Bioabbau der Verbindungen besonders vorteilhaft zur Geltung, zumal im allgemeinen auch auf den Zusatz von organischen Lösungsmitteln und Emulgatoren verzichtet werden kann. Mittel auf Basis dieser Alkylglykoside eignen sich daher insbesondere auch zur Anwendung an Pflanzen, die ganz oder in Teilen zum Verzehr bestimmt sind, selbst wenn die Ernte kurz bevor steht.

Die Alkylglycoside lassen sich aber auch in Kombination mit anderen Wirkstoffen aus den Gruppen der Insektizide, der Fungizide, der Viruzide, der Akarizide, der Herbizide, der Wachstumsregulatoren, der Reifebeschleuniger, der Repellents, der Blattdünger und anderen einsetzen. Bevorzugt werden Kombinationen mit anderen Insektiziden, Fungiziden und Akariziden sowie mit Herbiziden, bei denen es nicht nur zu einer Addition der Einzelwirkungen sondern auch zu einer synergistischen Wirkungsverstärkung kommen kann. Die Anwendung in Kombination mit anderen Insektziden, Fungiziden und Akariziden besitzt weiterhin den Vorzug, einer Resistenzbildung bei den Pflanzenschädlingen entgegenzuwirken. Hier wiederum wird insbesondere für Mittel, die an Nahrungsmittelpflanzen eingesetzt werden sollen, die Kombination mit solchen Wirkstoffen bevorzugt, die, wie die Alkylglycoside, keine Toxizität gegenüber Warmblütern aufweisen, oder die sehr rasch in unschädliche Produkte umgewandelt werden.

In den älteren Patentanmeldungen P 34 41 587.4-41 und P 35 07 380.2-41 wird der Einsatz von Alkylglycosiden als Wachsemulgatoren in verdunstungshemmenden Zusatzmitteln beschrieben, die zur Anwendung in der Landwirtschaft bestimmt sind. Diese Verwendung ist nicht Gegenstand des vorliegenden Schutzrechts.

Die Herstellung der Alkylglycoside kann auf an sich bekannten Wegen erfolgen. In diesem Zusammenhang wird auf die U.S. Patentschriften 3 547 828, 3 707 535 und 3 839 318, die deutschen Offenlegungsschriften 1 905 523, 1 943 689, 2 036 472 und 3 001 064 sowie auf die europäische Patentanmeldung 77 167 verwiesen.

Eine für den technischen Maßstab bedeutsame Synthese besteht in der säurekatalysierten Kondensation von Monosacchariden vom Typ der Aldosen H(-Glyk)H mit langkettigen primären Alkoholen (R-OH), die 8 bis 22, vorzugsweise 8 - 18 C-Atome enthalten. Unter Wasseraustritt entstehen Alkylglycoside der Formel II

$H(\text{-Glyk})_n\text{-R}$ (II),

wobei der Wert von n durch die Wahl der Reaktionsbedingungen in weiten Grenzen variiert werden kann.

Erfindungsgemäß brauchbar sind Alkylglycoside der Formeln I und II mit n = 1 bis 6, bevorzugt werden Verbindungen mit Werten für n zwischen 1 und 3. In Produkten, bei denen n größer als 1 ist, stellt n naturgemäß einen statistischen Mittelwert dar. Die Alkylglycoside der Formel I erhält man aus II durch Addition von Ethylenoxid, vorzugsweise unter alkalischer Katalyse, beispielsweise nach US-PS 2 407 002. Bevorzugt werden die Alkylglycoside mit 0 bis 10 Mol Ethylenoxid, wobei Ethylenoxid insbesondere auch völlig fehlen kann.

Bei der Herstellung der Alkylglycoside kann man auch von Oligo-oder Polysacchariden ausgehen, die dann im Verlauf der säurekatalysierten Reaktion zunächst durch Hydrolyse und/oder Alkoholyse zu niederen Bruchstücken depolymerisiert werden ehe sich die Alkylglycoside der Formel II bilden. Auch Gemische verschiedener reduzierender Monosaccharide oder Polysaccharide, die verschiedene Monosaccharideinheiten enthalten, lassen sich als Ausgangsmaterialien verwenden, wobei falls n größer als 1 ist, entsprechend gemischt zusammengesetzte Alkylglycosidmoleküle entstehen können.

Als Ausgangsmaterialien eignen sich folgende Monosaccharide:

Glucose, Mannose, Galaktose, Arabinose, Apiose, Lyxose, Gallose, Altrose, Idose, Ribose, Xylose und Talose sowie die aus diesen Monosacchariden zusammengesetzten Oligo- und Polysaccharide, beispielsweise Maltose, Lactose, Maltotriose, Hemicellulose, Stärke, Partialhydrolisate der Stärke und Zuckersirup.

Im Rahmen der Erfindung werden allerdings Alkylglycoside bevorzugt, die aus gleichen Monosaccharideinheiten aufgebaut sind. Besonders bevorzugt werden dabei Alkylglycoside, bei denen der Rest (-Glyk) von der Glucose abgeleitet ist. Für diese auch als Alkylglucoside bezeichneten Verbindungen werden entsprechend als Ausgangsmaterialien Glucose, Maltose, Stärke und andere Oligomere der Glucose verwendet.

Der Alkylteil R leitet sich bei der oben beschriebenen Herstellung von langkettigen, gegebenenfalls ungesättigten, primären Alkoholen ab, die verzweigt sein können, vorzugsweise aber nicht verzweigt sind. Beispiele sind die synthetischen Oxoalkohole mit 9 bis 15 C-Atomen und die aus natürlichen Fettsäuren gewonnenen Fettalkohole mit 8 - 22 C-Atomen. Besonders bevorzugt werden die Fettalkohole mit 8 bis 18 C-Atomen sowie ferner die Oxoalkohole mit 9 bis 15 C-Atomen.

Der Einsatz der Alkylglycoside im Pflanzenschutz erfolgt im allgemeinen in Form vorkonfektionierter Mittel, die die Alkylglycoside und gegebenenfalls weitere Wirkstoffe und Hilfsstoffe enthalten. Vor der Ausbringung werden diese Mittel in der Regel auf die Anwendungskonzentration verdünnt, doch ist es im Einzelfalle auch möglich, die Mittel bereits von Anfang an in der Anwendungskonzentration herzustellen. Übliche Verdünnungsgrade liegen zwischen 1 : 1 und 1 : 100, insbesondere 1 : 5 bis 1 : 50.

Bevorzugte Formulierungsform der Alkylglycoside ist die Lösung, wobei vor allem dann, wenn die Alkylglycoside als alleinige Wirkstoffe verwendet werden, vorzugsweise Wasser als Lösungsmittel dient.

Neben der Lösung kommen je nach Anwendungsgebiet der Mittel aber auch andere bei Pflanzenschutzmitteln übliche Zubereitungsformen, wie beispielsweise Emulsionen, Emulsionskonzentrate Suspensionen, Pasten, Pulver oder Granulate, in Betracht. Diese Formen besitzen Bedeutung vor allem dort, wo die Alkylglycoside in Kombination mit solchen Agrarchemikalien angewendet werden, die nicht als wäßrige Lösung konfektioniert werden.In vielen Fällen kann hier die Tensideigenschaft einiger Alkylglycoside zusätzlich zur Stabilisierung von Emulsionen oder Suspensionen genutzt werden.

Der Gehalt an Alkylglycosiden kann in den Mitteln im Extremfall bei 100 Gew.-% liegen, wird im allgemeinen aber, wegen der Mitverwendung von Hilfsstoffen und/oder anderen Wirkstoffen nicht über 90 Gew.-%, vorzugsweise nicht über 70 Gew.-% liegen. Bei wäßrigen Lösungen liegen die Gehalt in der Regel nicht über80 Gew.-%, insbesondere nicht über 70 Gew.-%. Die Untergrenzen liegen für Mittel, die ohne weitere Verdünnung angewendet werden, bei etwa 0,02 bis etwa 2 Gew.-%, vorzugsweise zwischen 0,1 und 1 Gew.-%. In den Mitteln, die vor Anwendung verdünnt werden, beträgt der Gehalt im allgemeinen mehr als 3 Gew.-%, vorzugsweise mehr als 5 Gew.-% und insbesondere mehr als 20 Gew.-%.

Als Hilfstoffe bei der Formulierung der alkylglycosidhaltigen Mittel kommen alle in Pflanzenschutzmitteln üblichen Hilfsstoffe, sofern sie mit den Alkylglycosiden verträglich sind, in Betracht. Als solche seien beispielsweise organische Lösungsmittel, feste unlösliche Trägerstoffe, Dispergiermittel, Emulgatoren, Viskositätsregulatoren und Streckmittel genannt.

Je nach Einsatzgebiet der Mittel kann die Anwendungskonzentration der Alkylglycoside in weiten Bereichen variiert werden. Die Untergrenze ist bei der Bekämpfung von Pflanzenschädlingen im allgemeinen erreicht, wenn bei vollständiger Benetzung der Pflanze gerade die für die beabsichtigte Wirkung notwendige Menge an Alkylglycosiden aufgebracht wird. Sie liegt, abhängig von den jeweils zu bekämpfenden Schädlingen, bei etwa 0,02 bis etwa 2 Gewichtsprozent, insbesondere zwischen 0,1 und 1 Gew.-%, kann aber in Extremfällen auch deutlich über oder unter diesen Werten liegen. Die Obergrenze der Anwendungskonzentration wird weitgehend durch die Löslichkeit der Alkylglycoside und die Viskosität der Lösungen bestimmt. In vielen Fällen lassen sich wäßrige Lösungen mit einem Gehalt von 70 Gew.-%

Alkylglycosid noch gut herstellen und handhaben. Dies ist vor allem bei gewerblicher Anwendung von Bedeutung, bei der großer Wert auf Gewichtseinsparung gelegt wird.

## Beispiele

1. Herstellung von Alkylglycosiden der Formel II (Tabelle 1)

Die Herstellung der Alkylglykoside A - D, F und G erfolgte durch säurekatalysierte Umsetzung von Glucose mit Fettalkoholen der Kettenlängen $C_8$ bis $C_{18}$ analog US-PS 3 839 318 (Methode 1). Die Alkylglucoside E und H wurden ausgehend von Butylglucosid durch Transglykosidierung analog US-PS 3 547 828 gewonnen (Methode 2). Mit Ausnahme von H, das zunächst durch Umkristallisieren aus Aceton weiter gereinigt wurde, wurden alle Produkte in technischer Reinheit zur Herstellung der erfindungsgemäßen Mittel eingesetzt.

Zusammensetzung und analytische Daten der Produkte sind in Tabelle 1 aufgeführt.

### Tabelle 1

Alkylglucoside A - H

| Chiffre | R | Herstellmethode | n* | OH-Zahl |
|---|---|---|---|---|
| A | n-Alkyl $C_8/C_{10}$ | 1 | 1,8 | 777 |
| B | n-Alkyl $C_8/C_{10}$ | 1 | 1,3 | 722 |
| C | n-Alkyl $C_{12}/C_{14}$ | 1 | 1,4 | 661 |
| D | n-Alkyl $C_{12}/C_{14}$ | 1 | 2,2 | 676 |
| E | Talgalkyl ($C_{16}/C_{18}$, partiell ungesättigt) | 2 | 5,4 | 804 |
| F | n-Alkyl $C_8/C_{10}$ | 1 | 1 | 709 |
| G | n-Alkyl $C_{12}/C_{14}$ | 1 | 1 | 590 |
| H | n-Alkyl $C_{12}$ | 2 | 1 | 638 |

* laut $^1H$ - NMR und Hydrolyseergebnis

2. Herstellung von Alkylglycosiden der Formel I

Die Darstellung der ethoxylierten Alkylglucoside I und K erfolgte analog US-PS 2 407 002 durch basenkatalysierte Anlagerung von stöchiometrischen Mengen Ethylenoxid an das Alkylglucosid G. Die Rohprodukte lagen als braune, viskose Massen vor und wurden durch Zusatz von 1 % $H_2O_2$ (70 %ige Lösung) und Erhitzen auf 100 °C gebleicht, ehe sie zur Herstellung der Mittel eingesetzt wurden.

Die beiden Verbindungen wiesen folgende Daten auf:

| Chiffre | R | n | m | OH-Zahl |
|---|---|---|---|---|
| I | n-Alkyl $C_{12}/C_{14}$ | 1 | 2 | 507 |
| K | n-Alkyl $C_{12}/C_{14}$ | 1 | 5 | 413 |

3. Herstellung der Pflanzenschutzmittel

Aus den Verbindungen A bis K wurden Mittel in Form wäßriger oder wäßrig-isopropanolischer Lösungen hergestellt, die unverdünnt oder mit Wasser verdünnt zur Anwendung kamen.

| Chiffre | Wirkstoff | Konzentration Gew.-% | Lösungsmittel |
|---|---|---|---|
| A 30 | A | 30 | Wasser |
| B 60 | B | 60 | Wasser |
| C 2 | C | 2 | Wasser |
| D 6 | D | 6 | Wasser |
| E 10 | E | 10 | Wasser |
| F 40 | F | 40 | Wasser |
| G 4 | G | 4 | Wasser |
| H 2 | H | 2 | Wasser |
| I 10 | I | 10 | Wasser/Isopropanol(85:15) |
| K 25 | K | 25 | Wasser/Isopropanol(85:15) |

4. Einsatz gegen Blattläuse Zur Bekämpfung von Blattläusen wurden Solanum-Jungpflanzen mit Blattläusen (Myzodes persicae) infiziert. Nachdem ein Befall von 100 - 150 Blattläusen auf den Pflanzen vorlag, wurden diese bis zur vollständigen Benetzung mit den Lösungen behandelt. Je 5 Pflanzen wurden für eine Versuchsreihe herangezogen. Die Kontrollen erfolgten, wie in der Tabelle 2 angegeben, 1, 3 bzw. 7 Tage nach der Behandlung. Die Leistung der Präparate wurde als Wirkungsgrad (Wg) nach der Abbott-Formel berechnet:

$$\text{Wg in \%} = \frac{\text{Lebende in der Kontrolle} - \text{Lebende im Versuch}}{\text{Lebende in der Kontrolle}} \times 100$$

5. Einsatz gegen Spinnmilben

Für die Versuche zur Bekämpfung von Spinnmilben wurden Buschbohnen angezogen und mit Spinnmilben (Tetranychus urticae) infiziert. Nachdem sich ein Befall von 10 - 15 Spinnmilben pro 10 $cm^2$ Blattfläche etabliert hatte, wurden die Pflanzen mit den Lösungen bis zur vollständigen Benetzung behandelt (je 5 Pflanzen wurden für eine Versuchsreihe herangezogen). Die Kontrollen der Pflanzen erfolgten 3, 7 bzw. 14 Tage, wie in der Tabelle 2 angegeben, nach der Behandlung. Der Wirkungsgrad (Wg) der Prüfprodukte wurde hier nach der Formel von Hendersen/Tilton berechnet:

$$\text{Wg in \%} = \left(1 - \frac{Bn \times Uv}{Bv \times Un}\right) \times 100$$

Bn = Zahl der Milben pro 10 $cm^2$ auf den behandelten Pflanzen nachher
Uv = Zahl der Milben pro 10 $cm^2$ auf den unbehandelten Pflanzen vorher
Bv = Zahl der Milben pro 10 $cm^2$ auf den behandelten Pflanzen vorher
Un = Zahl der Milben pro 10 $cm^2$ auf den unbehandelten Pflanzen nachher

Tabelle 2

Einsatz gegen Blattläuse und Spinnmilben

| Mittel | Wirkstoff | Anwendungskonzentration des Wirkstoffs Gew.-% | Blattläuse Wg in % nach Tagen | | | Spinnmilben Wg in % nach Tagen | | |
|---|---|---|---|---|---|---|---|---|
| | | | 1 | 3 | 7 | 3 | 7 | 14 |
| A 30 | A | 2,0 | 97,9 | 98,6 | 85,5 | 75,7 | 95,8 | 96,8 |
| B 60 | B | 2,0 | 96,8 | 97,5 | 96,1 | 63,7 | 100 | ---- |
| B 60 | B | 0,2 | ---- | ---- | ---- | 49,9 | 89,8 | ---- |
| C 2 | C | 0,2 | 98,4 | 91,4 | 73,9 | ---- | ---- | ---- |
| D 6 | D | 0,2 | 91,4 | 58,0 | 45,1 | ---- | ---- | ---- |
| E 10 | E | 2,0 | 92,2 | ---- | ---- | ---- | ---- | ---- |

6. Einsatz gegen Mehltau

Die Prüfung der Mittel gegen Echten Mehltau erfolgte an Elatior Begonien (Jungpflanzen), die mit dem Krankheitserreger Echter Mehltau (Oidium begoniae) infiziert wurden. Der Befallsgrad bis zur Anwendung der Lösungen bzw. nach Anwendung wurde in Wertzahlen von 1 - 9 nach folgender Tabelle ermittelt:

Wirkung des Mittels auf den Befall

1 = kein Befall
2 = sehr schwacher Befall bis 2,5 %
3 = schwacher Befall 2,5 bis 5,0 %
4 = noch schwacher Befall 5,0 bis 10,0 %
5 = mittelstarker Befall 10,0 bis 15,0 %
6 = starker Befall 15,0 bis 25,0 %
7 = sehr starker Befall 25,0 bis 35,0 %
8 = sehr starker Befall 35,0 bis 67,5 %
9 = Totalbefall 67,5 bis 100,0 %

Für jede Prüflösung wurden jeweils 5 Pflanzen verwendet, die bei den Versuchsreihen der Tabelle 3 bereits einen Befall vor Versuchsbeginn aufwiesen, während für die Versuchsreihen der Tabelle 4 Pflanzen ohne Befall eingesetzt wurden.

Die befallenen Pflanzen wurden 2 mal im Abstand von 14 Tagen bis zur vollständigen Benetzung besprüht und 7, 14 und 21 Tage nach der ersten Behandlung begutachtet. Tabelle 3 enthält die Bewertungszahlen nach 21 Tagen und den Durchschnitt aller drei Bewertungszahlen.

Die nicht befallenen Pflanzen wurden zunächst mit den Testlösungen behandelt, nach dem Abtrocknen mit Mehltau infiziert und nach 14 Tagen ein zweites Mal behandelt. Die Begutachtung der Wirkung erfolgte 7, 14 und 21 Tage nach der Infektion. Tabelle 4 zeigt die Ergebnisse in Form der Bewertungszahlen nach 21 Tagen und der Durchschnittswerte aller drei Bewertungen.

Tabelle 3

Einsatz gegen echte Mehltaupilze

2 Behandlungen im Abstand von 14 Tagen, Befall nach Wertzahlen 1 - 9

| Mittel | Wirkstoff | Anwendungs-konzentration des Wirkstoffs Gew.-% | Versuchsbeginn | Versuchsende | Ø aller Bewertungen |
|---|---|---|---|---|---|
| A 30 | A | 2,0 | 2,6 | 1,2 | 1,5 |
| B 60 | B | 0,2 | 2,2 | 1,6 | 1,9 |
| B 60 | B | 2,0 | 2,4 | 1,8 | 1,8 |
| F 40 | F | 0,2 | 2,4 | 2,5 | 2,4 |
| F 40 | F | 2,0 | 2,4 | 1,6 | 1,5 |
| G 4 | G | 0,2 | 2,4 | 2,2 | 1,9 |
| G 4 | G | 2,0 | 2,6 | 1,0 | 1,5 |
| I 10 | I | 2,0 | 2,8 | 1,2 | 1,5 |
| H 2 | H | 2,0 | 3,0 | 1,0 | 1,8 |
| | Unbehandelt | | 2,8 | 5,5 | 5,6 |

Tabelle 4

Einsatz gegen echte Mehltaupilze

Anwendung vorbeugend : Behandlung, Infektion, zweite Behandlung 14 Tage nach der ersten

Befall in Wertzahlen 1 - 9

| Mittel | Wirkstoff | Anwendungskonzentration des Wirkstoffs Gew.-% | Versuchsbeginn | Versuchsende | Ø aller Kontrollen |
|---|---|---|---|---|---|
| B 60 | B | 0,2 | 1 | 1,8 | 1,2 |
| B 60 | B | 2,0 | 1 | 1,8 | 1,2 |
| F 40 | F | 2,0 | 1 | 2,0 | 1,3 |
| I 10 | I | 2,0 | 1 | 1,4 | 1,3 |
| K 25 | K | 0,2 | 1 | 1,2 | 1,1 |
| K 25 | K | 2,0 | 1 | 1,2 | 1,1 |
| H 2 | H | 2,0 | 1 | 1,6 | 1,1 |
| Unbehandelt | | | 1 | 3,8 | 3,2 |

## Ansprüche

**1.** Verwendung von Alkylglycosiden der allgemeinen Formel

$H(-Glyk)_n-R \times (EO)_m$ (I)

in der (-Glyk) den Rest eines Monosaccharids, n einen Wert zwischen 1 und 6, R eine langkettige, primäre, glykosidisch gebundene Alkyl- oder Alkenylgruppe mit 8 - 22 C-Atomen, EO die Ethylenglykolethergruppe und m einen Wert von 0 bis 100 darstellen, als Schadorganismen bekämpfenden Wirkstoff in Mitteln zum Schutz von Nutz- und Zierpflanzen.

2. Verwendung nach Anspruch 1 von Alkylglykosiden gemäß Formel I in der der Rest (-Glyk) von Glucose abgeleitet ist.

3. Verwendung nach Anspruch 1 oder 2 von Alkylglycosiden gemäß Formel I, in der n einen Wert zwischen 1 und 3 besitzt.

4. Verwendung nach einem der Ansprüche 1 bis 3 von Alkylglycosiden gemäß Formel I, in der R ein unverzweigter primärer Alkyl- oder Alkenylrest mit 8 bis 18 C-Atomen ist.

5. Verwendung nach einem der Ansprüche 1 bis 4 von Alkylglycosiden gemäß Formel I, in der m einen Wert von 0 bis 10 besitzt.

6. Verwendung nach Anspruch 5, wobei m den Wert 0 hat.

7. Verwendung nach einem der Ansprüche 1 bis 6, bei der die Mittel mehr als 3 Gew.-%, vorzugsweise mehr als 5 Gew.-% und nicht mehr als 90 Gew.-%, vorzugsweise nicht mehr als 70 Gew.-% an Alkylglycosiden enthalten.

8. Verwendung nach einem der Ansprüche 1 bis 6, bei der die Mittel die Form wäßriger Lösungen haben und Alkylglycosidgehalte von 0,02 bis 70 Gew.% aufweisen.

9. Verwendung nach einem der Ansprüche 1 bis 8, bei der die Mittel neben den Alkylglycosiden wenigstens einen anderen Wirkstoff aus der Gruppe der Insektizide, Fungizide, Akarizide und Herbizide enthalten.

10. Verwendung nach einem der Ansprüche 1 bis 8, bei der die Alkylglycoside als alleinige Wirkstoffe verwendet werden.

11. Verwendung nach Anspruch 10 in Mitteln, die zum Gebrauch an Nahrungsmittelpflanzen bestimmt sind.

## Claims

1. The use of alkyl glycosides corresponding to the following general formula

$$H(-Glyc)_n-R\ x(EO)_m \qquad (I)$$

in which (-Glyc) is the residue of a monosaccharide, n has a value of from 1 to 6, R is a long-chain primary $C_{8-22}$ alkyl or alkenyl group attached by a glycoside bond, EO is the ethylene glycol ether group and m has a value of from 0 to 100, as active substance for controlling harmful organisms in preparations for the protection of useful and ornamental plants.

2. The use claimed in claim 1 of alkyl glycosides corresponding to formula I in which the residue (-Glyc) is derived from glucose.

3. The use claimed in claim 1 or 2 of alkyl glycosides corresponding to formula I in which n has a value of from 1 to 3.

4. The use claimed in any of claims 1 to 3 of alkyl glycosides corresponding to formula I in which R is an unbranched primary alkyl or alkenyl radical containing from 8 to 18 carbon atoms.

5. The use claimed in any of claims 1 to 4 of alkyl glycosides corresponding to formula I in which m has a value of from 0 to 10.

6. The use claimed in claim 5, wherein m has the value 0.

7. The use claimed in any of claims 1 to 6, wherein the preparations contain more than 3% by weight and preferably more than 5% by weight and no more than 90% by weight and preferably no more than 70% by weight of alkyl glycosides.

8. The use claimed in any of claims 1 to 6, wherein the preparations are in the form of aqueous solutions and have alkyl glycoside contents of from 0.02 to 70% by weight.

9. The use claimed in any of claims 1 to 8, wherein the preparations contain - in addition to the alkyl glycosides - at least one other active substance from the group comprising insecticides, fungicides, acaricides and herbicides.

10. The use claimed in any of claims 1 to 8, wherein the alkyl glycosides are used as sole active substances. 11. The use claimed in claim 10 in preparations intended for use on food plants.

**Revendications**

1. Utilisation d'alkylglucosides de formule générale:

$$H(-Glyk)_n - R \ x \ (EO)_m \qquad (I)$$

dans laquelle (-Glyk) est le radical d'un monosaccharide, n une valeur comprise entre 1 et 6, R un groupe alkyle ou alcényle avec 8 - 22 atomes de carbone, primaire, à longue chaîne, lié à la partie glucosidique, EO est le groupe éthylèneglycoléther et m a une valeur de 0 à 100, comme principe actif contre des organismes nuisibles dans des agents de protection des plantes utiles et des plantes ornementales.

2. Utilisation d'alkylglucosides de formule I selon la revendication 1, où le radical (-Glyk) est dérivé du glucose.

3. Utilisation d'alkylglucosides de formule I selon la revendication 1 ou 2, où n a une valeur comprise entre 1 et 3.

4. Utilisation d'alkylglucosides de formule I selon l'une des revendications 1 à 3, où R est un radical alkyle ou alcényle primaire non ramifié, comportant 8 à 18 atomes de carbone.

5. Utilisation d'alkylglucosides de formule I selon l'une des revendications 1 à 4, où m a une valeur de 0 à 10.

6. Utilisation selon la revendication 5, dans laquelle m a la valeur 0.

7. Utilisation selon l'une des revendications 1 à 6, dans laquelle l'agent contient plus de 3 % en poids, de préférence plus de 5 % en poids, mais pas plus de 90 % en poids, de préférence pas plus de 70 % en poids d'alkylglucosides.

8. Utilisation selon l'une des revendications 1 à 6, dans lequelle les agents se présentent sous la forme de solutions aqueuses et présentent des teneurs en alkylglucosides de 0,02 à 70 % en poids.

9. Utilisation selon l'une des revendications 1 à 8 dans lequelle les agents contiennent, à côté des alkylglucosides, au moins un autre principe actif du groupe des insecticides, fongicides, acaricides et herbicides.

**10.** Utilisation selon l'une quelconque des revendications 1 à 8, dans laquelle les alkylglucosides sont utilisés comme principes actifs exclusifs.

**11.** Utilisation selon la revendication 10 dans des agents qui sont destinés à être utilisés sur des plantes alimentaires.